# EUROPEAN PATENT APPLICATION

(11) **EP 2 130 925 A1**
(43) Date of publication of application: **09.12.2009**
(21) Application number: 08712079.6
(22) Date of filing: 28.02.2008
(51) Int. Cl.: C12P 41/00

(54) **METHOD FOR PRODUCTION OF OPTICALLY ACTIVE AMINO ACID**

(30) Priority: 28.02.2007 JP 2007049301
(71) Applicant: Mitsubishi Gas Chemical Company, Inc., Chiyoda-ku Tokyo 100-8324 (JP)
(72) Inventor: ASANO, Yasuhisa, Toyama-shi Toyama 930-0066 (JP); TANAKA, Akinori, Niigata-shi Niigata 950-3112 (JP); HASEMI, Ryuji, Niigata-shi Niigata 950-3112 (JP)
(74) Representative: Harrison, Robert John
(86) International application number: PCT/JP2008/053508
(87) International publication number: WO 2008/105493

(57) **Abstract**

An optically active amino acid is useful as food or feed, agrochemicals, chemical products for industrial use, intermediates for synthesis of cosmetics or medicines and the like and is also important as optical resolving agents or chiral building blocks for use in organic synthesis. Thus, the object is to provide an industrially practical method for producing the optically active amino acid simply and at low cost. The method comprises the step of reacting an aminonitrile composed of a mixture of a D-aminonitrile and an L-aminonitrile with a biocatalyst which is one derived from a newly isolated microorganism belonging to the genus Rhodococcus and has an activity of converting the two aminonitriles into a D-amino acid amide and an L-amino acid amide respectively, a biocatalyst which has an activity of racemizing the D-amino acid amide and the L-amino acid amide to each other, and a biocatalyst which has an activity of converting one of the D-amino acid amide and the L-amino acid amide into the corresponding D- or L-amino acid.

## Description

### TECHNICAL FIELD

The present invention relates to a method for production of an optically active amino acid, that is, an amino acid in D-form or L-form. More specifically, it relates to a method for producing an optically active amino acid from an aminonitrile represented by the formula (1) using a biocatalyst derived from a microorganism. Furthermore specifically, it relates to a method for producing an optically active amino acid consisting of a D-amino acid or an L-amino acid from an aminonitrile represented by the formula (1), **characterized in that** the D-amino acid or L-amino acid is obtained by providing an aminonitrile composed of a mixture of a D-aminonitrile and an L-aminonitrile as a raw material, and reacting it with a biocatalyst which has an activity of converting the two aminonitriles into a D-amino acid amide and an L-amino acid amide respectively, a biocatalyst which has an activity of racemizing the D-amino acid amide and the L-amino acid amide to each other, and a biocatalyst which has an activity of converting one of the D-amino acid amide and the L-amino acid amide into the corresponding D- or L-amino acid.

The optically active amino acid is useful as food or feed, agrochemicals, chemical products for industrial use, intermediates for synthesis of cosmetics or medicines and the like, and is important as optical resolving agents or chiral building blocks for use in organic synthesis. 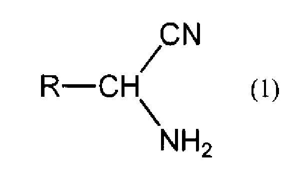 wherein R in the formula (1) is a straight or branched lower alkyl group with 1-4 carbon atoms, a phenyl group or a phenylmethyl group, and may have a hydroxyl group or methylmercapto group as a substituent.

### BACKGROUND ART

As methods for production of an optically active amino acid, many processes such as a fermentation process, a synthetic process and an enzymatic process have conventionally been known.

Since a racemic aminonitrile can be relatively easily synthesized as an intermediate in the Strecker amino acid synthesis, a method for producing an optically active amino acid from a racemic aminonitrile utilizing a biocatalyst derived from a microorganism has been reported, and the following methods have been known.

A method for preparing optically active amino acids and amino acid amides by reacting a racemic aminonitrile with an enantioselective aminonitrile hydratase is disclosed (for example, refers to Patent Document 1). This report discloses that an L-amino acid amide was obtained from a racemic aminonitrile, but its enantiomer-selectivity was low, and enantiomer excess of the resulting L-amino acid amide was merely 40% e.e. Also, in order to obtain the target L-amino acid amide, hydrolysis of the raw material L-amino acid amide must be effected with very complicated means that require enzymatic hydrolysis and chemical hydrolysis to be repeated 5 times, thereby making enantiomer excess of the resulting L-amino acid further lowered to 35% e.e.

A method for preparing an L-amino acid and a D-amino acid amide or a D-amino acid and an L-amino acid amide by reacting a biocatalyst derived from a microorganism with a racemic aminonitrile is disclosed (for example, refers to Patent Documents 2 and 3). However, in these methods, the amino acid and the amino acid amide are generated in a ratio of 1:1, and thus each yield does not exceed 50%, and in order to obtain a target L-form or D-form optically active amino acid, it is required to separate the two compounds by some means and change one of them into the target optically active amino acid.

A method for directly obtaining an L-amino acid by reacting a biocatalyst derived from a microorganism with a racemic aminonitrile is disclosed (for example, refers to Patent Documents 4 to 7). However, yield of L-amino acid resulting from the raw material racemic aminonitrile was about 35% at maximum in accordance with Examples and thus productivity was very low, possibly because the activity of the enzyme involved in the reaction was a very weak or the expressed amount of the enzyme was very small although detailed reasons are unknown.

A method for obtaining an L-amino acid by reacting a biocatalyst derived from a microorganism which belongs to the genus Acinetobacter with a racemic aminonitrile to convert it into a racemic amino acid amide, and further by reacting a microorganism having an amino acid amide racemase activity and an L-amino acid amide amidase activity therewith (a microorganism belonging to the genus Arthrobacter or Corynebacterium) is disclosed (for example, refers to Patent Document 8). If each enzymatic reaction in this method makes good progress, the L-amino acid should theoretically be obtained from the racemic aminonitrile in 100% yield. However, the yield of the resulting L- amino acid was as low as 65%, and this method is not so satisfactory in productivity as to be adopted industrially.

A method in which the whole cell of a microorganism containing a cloned gene for nitrile hydratase, a cloned gene for amidase or D-amidase and a cloned gene for amino acid amide racemase is used as a catalyst is disclosed (for example, refers to Patent Document 9). However, there is no description at all about a concrete preparation method of the whole cell catalyst and a concrete method or working example for production of an optically active amino acid from a racemic aminonitrile using the whole cell catalyst.

There has been a description about obtaining a D-amino acid or an L-amino acid from a racemic amino acid amide by use of an α-amino-ε-caprolactam racemase in combination with a D-form selective hydrolysis enzyme or an L-form selective hydrolysis enzyme (for example, refers to Non-Patent Document 1). After a racemic aminonitrile is chemically converted into a racemic amino acid amide, it can be used in the method mentioned in the above Non-Patent Document 1. However, when this method is actually practiced industrially, problems occur such that enzymatic reaction may be considerably inhibited or purity of the resulting amino acid may be lowered, if the chemical-amidation reaction solution is used as it is without purification, or there will be a great loss if purification step is required in order to improve the purity, and consequently the overall isolated yield may be lowered. On the other hand, if the racemic amino acid amide is used in the method mentioned in the above Non-Patent Document 1 after isolated and purified from the chemical amidation reaction solution, the enzymatic reaction inhibition will be lowered, but there will be a great loss in this step, and consequently the overall isolated yield will be lowered. Also, when a solution containing a free amino acid amide or a free amino acid amide itself is stored for a long time, it is liable to convert into an amino acid gradually by hydrolysis so that a quality problem may occur such as decrease in optical purity of amino acids resulting from the enzymatic reaction. As mentioned above, the method of chemically converting the racemic aminonitrile into the amino acid amide still has many problems to be solved for the industrial practice, such that the reaction cannot be performed in one pot, complicated operation, apparatus and time are required for crystallization and separation, solvent collection and the like after hydrolysis reaction, and also environmental impact occurs due to the use of alkali catalysts and metallic catalysts, or the use of organic solvents such as alcohol and acetone.
Non-Patent Document 1: Yasuhisa Asano, J. Mol. Catal. B: Enzymatic 36, 22-29, 2005.
Patent Document 1: JP-A-S63-500004
Patent Document 2: JP-B-H03-16118
Patent Document 3: JP-A-H02-31694
Patent Document 4: JP-B-H07-20432
Patent Document 5: JP-B-H07-24590
Patent Document 6: JP-B-2670838
Patent Document 7: JP-B-2864277
Patent Document 8: JP-A-H03-500484
Patent Document 9: JP-A-2003-225094

### DISCLOSURE OF THE INVENTION

### PROBLEMS TO BE SOLVED BY THE INVENTION

The present invention aims at solving the problems as described above in the conventional techniques and providing an industrially practical method which can simply and economically produce an optically active amino acid useful as food or feed, agrochemicals, chemical products for industrial use, intermediates for synthesis of cosmetics or medicines and the like and also important as optical resolving agents or chiral building blocks for use in organic synthesis.

### MEANS FOR SOLVING THE PROBLEM

The present inventors have studied intensively in order to establish a method for producing an optically active amino acid in a simple way at good yield, and consequently have found that an optically active substance, namely, a D-amino acid or an L-amino acid can effectively be produced by providing a mixture of a D-aminonitrile and an L-aminonitrile represented by the formula (1) as a raw material, and reacting it with a biocatalyst which has an activity of converting the two aminonitriles into a D-amino acid amide and an L-amino acid amide, a biocatalyst which has an activity of racemizing the D-amino acid amide and the L-amino acid amide, and a biocatalyst which has an activity of selectively reacting with either the D-amino acid amide or the L-amino acid amide to convert it into the corresponding D- or L-amino acid.

That is, searches have extensively been made throughout the natural world in order to obtain a biocatalyst that has an activity of converting a mixture of a D-aminonitrile and an L-aminonitrile into a D-amino acid amide and an L-amino acid amide, and particularly exhibits the activity at a high level even in the same enzymatic reaction system and condition as a biocatalyst which has an activity of racemizing the D-amino acid amide and the L-amino acid amide and a biocatalyst which has an activity of selectively reacting with either the D-amino acid amide or the L-amino acid amide so as to convert it into a amino acid. As a result, it has been found that a microorganism belonging to the genus Rhodococcus, concretely Rhodococcus opacus 71D (FERM AP-21233 or International deposit No. FERM BP-10952) is a very preferable biocatalyst from the viewpoint of activity and substrate specificity. Meanwhile, this microorganism has been deposited in International Patent Organism Depositary (IPOD), National Institute of Advanced Industrial Science and Technology (AIST) on February 27, 2007. This microorganism is high in the activity of converting both D- and L- forms of aminonitrile into the corresponding amino acid amides but has substantially no activity of causing another reaction, for example, hydrolyzing the generated amino acid amides so as to further convert them into D- or L-form of amino acid. Therefore, the present strain has an ability to convert the racemic aminonitrile into the racemic amino acid amide with high selectivity and yield, for example. Also, this microorganism is characteristic in that it exhibits a high activity in the same enzymatic reaction condition as the concurrently used biocatalysts, namely, a biocatalyst which racemizes a D-amino acid amide and an L-amino acid amide and a biocatalyst which has an activity of selectively reacting with either the D-amino acid amide or the L-amino acid amide so as to convert it into the corresponding amino acid. Thus, it has been found that this microorganism can extremely preferably be used in the method of the present invention, and the present invention has been completed.

That is, the present invention relates to a method for producing an optically active amino acid from an aminonitrile by combined use of biocatalysts including one derived from a newly-isolated microorganism which belongs to the genus Rhodococcus (Rhodococcus opacus 71D), as defined in the followings (1) to (10).
(1) A method for producing an optically active amino acid composed of a D- or an L-amino acid, which comprises reacting an aminonitrile composed of a mixture of a D-aminonitrile and an L-aminonitrile represented by formula (1) with a biocatalyst which has an activity of converting the two aminonitriles into a D-amino acid amide and an L-amino acid amide respectively, a biocatalyst which has an activity of racemizing the D-amino acid amide and the L-amino acid amide to each other, and a biocatalyst which has an activity of converting one of the D-amino acid amide and the L-amino acid amide into the corresponding D- or L-amino acid. 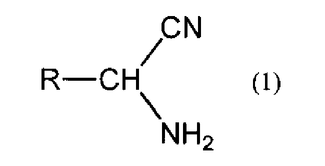 wherein R in the formula (1) is a straight or branched lower alkyl group with 1-4 carbon atoms, a phenyl group or a phenylmethyl group, and may have a hydroxyl group or methylmercapto group as a substituent.
(2) The method for producing an optically active amino acid according to (1), wherein said biocatalyst having an activity of converting the D- and L-aminonitriles into a D-amino acid amide and an L-amino acid amide respectively is one derived from a microorganism belonging to the genus Rhodococcus.
(3) The method for producing an optically active amino acid according to (1), wherein said biocatalyst having an activity of converting the D- and L-aminonitriles into a D-amino acid amide and an L-amino acid amide respectively is one derived from Rhodococcus opacus.
(4) The method for producing an optically active amino acid according to (1), wherein said biocatalyst having an activity of racemizing the D-amino acid amide and the L-amino acid amide to each other is one derived from a microorganism belonging to the genus Achromobacter.
(5) The method for producing an optically active amino acid according to (1), wherein said biocatalyst having an activity of racemizing the D-amino acid amide and the L-amino acid amide to each other is one derived from Achromobacter obae.
(6) The method for producing an optically active amino acid according to (1), wherein said biocatalyst having an activity of converting the D-amino acid amide selected from the D-amino acid amide and the L-amino acid amide into the corresponding D-amino acid is one derived from a microorganism belonging to the genus Ochrobactrum.
(7) The method for producing an optically active amino acid according to (1), wherein said biocatalyst having an activity of converting the D-amino acid amide selected from the D-amino acid amide and the L-amino acid amide into the corresponding D-amino acid is one derived from Ochrobactrum anthropi.
(8) The method for producing an optically active amino acid according to (1), wherein said biocatalyst having an activity of converting the L-amino acid amide selected from the D-amino acid amide and the L-amino acid amide into the corresponding L-amino acid is one derived from a microorganism belonging to the genus Brevundimonas or the genus Xanthobacter.
(9) The method for producing an optically active amino acid according to (1), wherein said biocatalyst having an activity of converting the L-amino acid amide selected from the D-amino acid amide and the L-amino acid amide into the corresponding L-amino acid is one derived from Brevundimonas diminuta or Xanthobacter flavus.
(10) The method for producing an optically active amino acid according to (1), wherein, as biocatalysts, those derived from Rhodococcus opacus and Achromobacter obae and one derived from Ochrobactrum anthropi or Brevundimonas diminuta are used in combination.

### EFFECT OF THE INVENTION

According to the method of the present invention, an optically active amino acid can be produced readily in one pot, which is useful as food or feed, agrochemicals, chemical products for industrial use, intermediates for synthesis of cosmetics or medicines and the like, and is important as optical resolving agents or chiral building blocks for use in organic synthesis.

### BEST MODE FOR CARRYING OUT THE INVENTION

Hereinafter, the best mode for carrying out the present invention will be described in detail.

The raw material of the present invention only has to be a mixture of a D-aminonitrile and an L-aminonitrile represented by formula (1), and the production method thereof is not specifically limited. Usually, it can be obtained as an intermediate of the amino acid synthesis by the Strecker reaction in which an aldehyde is used as a starting material.

Concretely, it can be synthesized with a method of reacting an aldehyde with hydrogen cyanide and ammonia or first synthesizing a cyanhydrin from an aldehyde and hydrogen cyanide and then reacting it with ammonia, but aminonitriles are extremely unstable and thus are problematic in handling such that they are gradually colored in reddish brown and finally turn black to generate tar-like substances even when they are stored below room temperature. Also, it is problematic in that the enzymatic reaction is inhibited by impurities such as a very small amount of hydrogen cyanide contaminating the reaction solution. It might be possible to purify aminonitriles as a free form by methods such as crystallization and recrystallization, but aminonitriles are usually difficult to crystallize and are isolated and recovered in low yield from the reaction solution.

That is, these problems can be avoided by adding an acid to the resulting aminonitrile to isolate it as a salt, so that the present invention can more easily be practiced. Examples of the acid to be added include a mineral acid such as hydrochloric acid and sulfuric acid and an organic acid such as acetic acid, but hydrochloric acid and sulfuric acid are particularly preferably used considering ease of crystallization or handling the salt and the cost totally.

R in the structural formula of aminonitrile represented by the formula (1) is determined by three kinds of biocatalyst used in combination in the present invention, that is, depends upon a structure of a substrate to which all the three kinds of biocatalyst show high reactivity. As a result of carefully examining the substrate specificity of the three kinds of biocatalyst in the same reaction condition, it has been found that examples of R include methyl group, ethyl group, n-propyl group, isopropyl group, n-butyl group, isobutyl group, t-butyl group, hydroxymethyl group, 1-hydroxyethyl group, 2-methylmercaptoethyl group, phenyl group and phenylmethyl group, and particularly preferably are methyl group and ethyl group.

In the present invention, a biocatalyst having an activity of converting a mixture of a D-aminonitrile and an L-aminonitrile into a D-amino acid amide and an L-amino acid amide, a biocatalyst which has an activity of racemizing the D-amino acid amide and the L-amino acid amide, and a biocatalyst which has an activity of converting one of the D-amino acid amide and the L-amino acid amide into the corresponding amino acid are used.

Here, a biocatalyst having an activity means microbial cells or processed products of microbial cells, and examples of the processed products of microbial cells include acetone powders, partially purified enzymes, purified enzymes, and immobilized enzymes which comprise immobilized microbial cells or purified enzymes.

A biocatalyst derived from a microorganism means not only microbial cells of the microorganism or processed products of such microbial cells but also microbial cells of a transformant into which a gene coding an enzyme of the microorganism is incorporated or processed products of such microbial cells.

Searches have extensively been made in the natural world in order to obtain a biocatalyst which has an activity of converting a mixture of a D-aminonitrile and an L-aminonitrile into a D-amino acid amide and an L-amino acid amide, and particularly exhibits a high activity even in the same condition as a biocatalyst which has an activity of racemizing the D-amino acid amide and the L-amino acid amide and a biocatalyst which has an activity of selectively reacting with the D-amino acid or the L-amino acid so as to convert it into an amino acid. As a result, it has been found that a microorganism belong to the genus Rhodococcus, specifically Rhodococcus opacus 71D (FERM AP-21233 or International deposit No. FERM BP-10952) is very preferable from the viewpoint of activity and substrate specificity. Meanwhile, this microorganism was deposited in International Patent Organism Depository (IPOD), National Institute of Advanced Industrial Science and Technology (AIST) (Tsukuba Central 6, 1-1-1 Higashi, Tsukuba, Ibaraki, Japan, Postal code 305-8566) on February 27, 2007, and then transferred to international depository of the same center on February 18, 2008. This microorganism is low in stereoselectivity for conversion into an amino acid amide and is also low in enzymatic activity involving reactions other than this, and has an excellent property of converting a racemic aminonitrile into a racemic amino acid amide at high yield. Also, this microorganism exhibits a high activity in the same enzymatic reaction condition as a biocatalyst which racemizes a D-amino acid amide and an L-amino acid amide and a biocatalyst which has an ability to selectively react with the D-amino acid amide or the L-amino acid amide so as to convert it into the corresponding amino acid, and thus can especially suitably be used in the method of the present invention.

As mentioned above, since this microorganism is, for example, high in the biocatalytic activity of converting a racemic aminonitrile into a racemic amino acid amide, and very low in the other activities, for example, an activity of converting the amino acid amide into the amino acid, it is substantially not observed that the generated amino acid amide is further hydrolyzed by the enzyme of this microorganism. Therefore, it has become possible to arbitrarily produce either one of the D-amino acid and the L-amino acid quantitatively by use of microbial cells or a partially-purified or purified enzyme of this microorganism in combination with the other biocatalysts, that is, a biocatalyst having an activity of racemizing a D-amino acid amide and an L-amino acid amide and a biocatalyst having an activity of selectively reacting with the D-amino acid amide or the L-amino acid amide to convert it into the corresponding optically active amino acid. Concretely, by use of this microorganism in combination with a biocatalyst having the activity of racemizing the D-amino acid amide and the L-amino acid amide and a biocatalyst having the activity of selectively reacting with the D-amino acid amide to convert it into the D-amino acid, only the D-amino acid can be produced from the racemic aminonitrile. Also, by use of this microorganism in combination with a biocatalyst having the activity of racemizing the D-amino acid amide and the L-amino acid amide and a biocatalyst having the activity of selectively reacting with the L-amino acid amide to convert it into the L-amino acid, only the L-amino acid can be produced from the racemic aminonitrile.

Because this microorganism has the characteristic property of being high in the activity of converting a racemic aminonitrile into a racemic amino acid amide and being very low in the other activities, for example, the activity of converting the amino acid amide into an amino acid, it can provide an optically active amino acid high in optical purity when it is used in combination with a biocatalyst high in the activity of selectively reacting with a D-amino acid amide or an L-amino acid amide to convert it into an amino acid.

Particularly, referring to substrate specificity, it shows a high activity against a wide range of substrates from a substrate having an aliphatic substituent with a few carbon atoms, concretely α-aminobutyronitrile, to a substrate having an aromatic ring substituent, concretely phenylglycinonitrile, which is said to be a suitable property when used in combination with the other biocatalysts.

The taxonomic characters of this microorganism are shown as follows. Gram stain: +, shape: rod, acid-fast: -, motility: -, presence or absence of spore: -, aerobe: +, anaerobe: -, catalase: +, oxidase: -, glucose: -, OF test: -/-, reduction of nitrate: -, denitrification: -, MR reaction: -, VP reaction: -, production of indole: -, production of H₂S: -, starch hydrolysis: +, citric acid utilization: +, ammonium sulfate utilization: -, production of pigment: -, urease: +, production of dihydroxyacetone: -, cellulose hydrolysis: -, malonic acid utilization: -, 5%NaCl: -, DNase: -, Tween hydrolysis: -, requirement for vitamins: -, gelatin liquefaction: -, n-hexadecane utilization: +, litmus milk: -, growth temperature: 30°C, growth pH: 6-9.

As mentioned above, since this microorganism is a gram-positive nonmotile rod-shaped bacterium which does not form spore, is positive in catalase reaction and is positive in oxidase reaction, it is judged as a microorganism belonging to the genus Rhodococcus. Further, when a taxon was supposed by a partial nucleotide sequence of 16SrDNA (16SrRNA gene), it corresponded with Rhodococcus opacus at high homology, and thus has been identified as Rhodococcus opacus, and named as 71D strain.

A biocatalyst involved in an activity of converting a racemic aminonitrile into a racemic amino acid amide, which is derived from the microorganism, was purified with a variety of known purification methods such as ammonium sulfate fractionation, butyl-TOYOPEARL, DEAE-TOYOPEARL, Gigapite, mono Q and hydroxyapatite in combination, and the results of investigation of enzymatic properties thereof are shown in Table 1. Experimental procedures are described in the following Examples.

### Results for the enzyme purification

| | Total protein (mg) | Total activity (unit) | Specific activity (unit/mg) | Yield (%) |
|---|---|---|---|---|
| Cell-free extract | 1840 | 7500 | 4.07 | 100 |
| Ammonium sulfate fractionation | 1170 | 6200 | 5.28 | 82.6 |
| DEAE-Toyopearl | 131 | 5020 | 38.3 | 66.9 |
| Butyl-Toyopearl | 25.1 | 3510 | 140 | 46.8 |
| Gigapite | 16.7 | 3490 | 209 | 46.6 |
| Mono Q | 2.29 | 2140 | 934 | 28.5 |
| Hydroxyapatite | 0.88 | 919 | 1040 | 12.3 |

### Enzymatic properties

Molecular weight: 119,000, subunit: (α) 27,200, (β) 32,300, subunit structure: α2β2, pH at which activity is exhibited: 4-11, optimum pH: 8, temperature at which activity is exhibited: up to 70°C, and optimum temperature: 20°C. Relative activity for each substrate is shown below supposing that the activity for 2-aminobutyronitrile is 100. Alaninonitrile 77, valinonitrile 15.1, leucinonitrile 11.8, t-leucinonitrile 1.80, phenylglycinonitrile 177, and phenylalaninonitrile 42.9.

An example of a biocatalyst having an activity of racemizing a D-amino acid amide and an L-amino acid amide includes Achromobacter obae disclosed in the Non-Patent Document 1, and an enzyme having the activity of racemizing the D-amino acid amide and the L-amino acid amide derived from this microorganism, and a transformant such as of Escherichia coli into which a gene coding this enzyme is incorporated can also be used. Properties of this enzyme are as follows; molecular weight: 51,000 (gel filtration), 45,568 (gene sequence), subunit: monomer, pH at which activity is exhibited: 5-10, optimum pH: 8.8, temperature at which activity is exhibited: up to 55°C. Relative activity for each substrate is shown below supposing that the activity for 2-aminobutyric acid amide is 100. L-alaninamide 78, L-threoninamide 63, L-norvalinamide 63, L-norleuicinamide 56, L-leuicinamide 48, L-methioninamide 35, L-serinamide 17, and L-phenylalaninamide 2.

An example of a microorganism having an activity of selectively reacting with a D-amino acid amide to convert it into an amino acid includes Ochrobactrum anthropi disclosed in the Non-Patent Document 2 (J. Biological Chemistry 264 (24), 14233-14239, 1989), and an enzyme having the activity of selectively converting the D-amino acid amide into the D-amino acid derived from this microorganism, and a transformant such as of Escherichia coli into which a gene coding this enzyme is incorporated (Non-Patent Document 3: Biochemistry, 31, 2316-2328, 1992) can also be used. Properties of this enzyme are as follows; molecular weight: 122000, subunit: 59000, subunit number 2, pH at which activity is exhibited: 5-10, optimum pH: 8.5, temperature at which activity is exhibited: up to 55°C, optimum temperature: 45°C. Relative activity for each substrate is shown below supposing that the activity for D-2-aminobutyric acid amide is 100. D-alaninamide 333, D-serinamide 97, D-threoninamide 30, D-methioninamide 6.7, D-norvalinamide 6, D-norleuicinamide 2.7, and D-phenylglycinamide 2.3.

An example of a microorganism having an activity of selectively reacting with an L-amino acid amide to convert it into an amino acid includes Brevundimonas diminuta disclosed in Non-Patent Document 4 (Appl. Microbiol. Biotechnol. 70, 412-421, 2006) and Xanthobacter flavus disclosed in Non-Patent Document 5 (Adv. Synth. Catal. 347, 1132-1138, 2005), and an enzyme having the activity of selectively converting the L-amino acid amide into the L-amino acid derived from this microorganism, and a transformant such as of Escherichia coli into which a gene coding this enzyme is incorporated can also be used. Properties of this enzyme in case of Brevundimonas diminuta are as follows; molecular weight: 288000, subunit: 53000, subunit number: 6, pH at which activity is exhibited: 5-10, optimum pH: 7.5, temperature at which activity is exhibited: up to 80°C, optimum temperature: 50°C. Relative activity for each substrate is shown below supposing that the activity for L-2-aminobutyric acid amide is 100. L-alaninamide 3.4, L-valinamide 17.7, L-leuicinamide 92.7, L-t-leuicinamide 0.2, L-isoleuicinamide 30.2, L-serinamide 2.9, L-threoninamide 14.6, L-methioninamide 85.4, and L-phenylalaninamide 104.

In case of Xanthobacter flavus, molecular weight: 38555, subunit number: 1, pH at which activity is exhibited: 4-10, optimum pH: 7.0, temperature at which activity is exhibited: up to 80°C, optimum temperature: 55°C. Relative activity for each substrate is shown below supposing that the activity for L-2-aminobutyric acid amide is 100. L-valinamide 65, L-t-leuicinamide 8.4, L-phenylglycinamide 113, and L-phenylalaninamide 111.

All of the biocatalyst having an activity of converting a mixture of a D-aminonitrile and an L-aminonitrile into a D-amino acid amide and an L-amino acid amide, the biocatalyst having an activity of racemizing the D-amino acid amide and the L-amino acid amide, and the biocatalyst having an activity of selectively reacting with the D-amino acid amide or the L-amino acid amide to convert it into an amino acid can be used in a form of microbial cells or processed products of microbial cells. Examples of processed products of microbial cells include a partially purified enzyme, a purified enzyme and acetone powder, and particularly acetone powder which is said to be a suitable form of use considering industrial application because it does not cause decomposition of microbial cells but can be stored in a small space with their activity being maintained for a long time. Acetone powder formation can be practiced by a known method disclosed in, for example, Non-Patent Document 6 (J. Org. Chem., 55. 5567-5571, 1990).

Also, microbial cells or purified enzymes can be used in a form immobilized with a known method such as entrapment immobilization and adsorption immobilization.

The present invention is characterized by the combined use of a biocatalyst having an activity of converting a mixture of a D-aminonitrile and an L-aminonitrile into a D-amino acid amide and an L-amino acid amide, a biocatalyst having an activity of racemizing the D-amino acid amide and the L-amino acid amide, and a biocatalyst having an activity of selectively reacting with the D-amino acid amide or the L-amino acid amide to convert it into an amino acid, and it is important to decide a reaction condition taking into account of properties of the respective biocatalysts, particularly pH and temperature at which activity is exhibited. The pH for the enzymatic reaction is outside the range of not more than 4 or not less than 10 where enzymatic activity is considerably inactivated, and the reaction can be performed, for example, in a range of pH 5-9, and particularly suitably in a range of pH 6-8.8. The reaction temperature is outside the range of not less than 80°C where enzymatic reaction is considerably inactivated or not more than 0°C where reaction rate is considerably lowered, and the reaction can be performed, for example, in a range of 5°C-60°C, and particularly suitably in a range of 20°C-45°C. When concentration of aminonitrile as a raw material is lowered, productivity per vessel is lowered, and thus a disadvantage arises considering industrial operation, and when the concentration becomes high, substrate inhibition or product inhibition occurs. Thus, the concentration actually employed is in a range of 0.1%-10% and preferably 0.1%-1%.

The amount to be used of each biocatalyst derived from a microorganism differs depending upon the activity per weight of the biocatalyst, and is difficult to define generally, but the object can be achieved by using an amount sufficient for completing each reaction in a desired reaction time. When industrially practiced, an activity per weight of a biocatalyst derived from a microorganism should be determined in advance in a small-scale preliminary examination, so that the amount sufficient for completing each reaction in a desired reaction time can be defined. A concrete amount to be used will be illustrated in the following Examples.

As a method for separating and purifying an optically active amino acid from a reaction solution after completion of the reaction, a known method can be used including condensation crystallization, solvent substitution and methods using ion-exchange resins after most of the microbial cells and the components derived from the cells have been removed by centrifugation, filtration or the like. According to the present invention, since aminonitrile as the raw material is converted almost quantitatively into the target optically active amino acid, it is almost unnecessary to separate the aminonitrile as the raw material and the amino acid amide as the intermediate from the target optically active amino acid. Thus, separation and purification of the target optically active amino acid is very easy, and this point also can be said to be advantageous for industrial practices.

The present invention enables simply producing an optically active amino acid which is useful as food or feed, agrochemicals, chemical products for industrial use, intermediates for synthesis of cosmetics or medicines and the like and is also important as optical resolving agents or chiral building blocks for use in organic synthesis, such as D-alanine, L-alanine, D-aminobutyric acid, L-aminobutyric acid, D-valine, L-valine, D-leuicine, L-leuicine, D-serine, L-serine, D-threonine, L-threonine, D-methionine, L-methionine, D-phenylglycine, L-phenylglycine, D-phenylalanine or L-phenylalanine, from a racemic 2-aminopropyonitrile, 2-aminobutyronitrile, 2-amino-3-methylbutyronitrile, 2-amino-4-methylpentanitrile, 2-amino-3-hydroxypropyonitrile, 2-amino-3-hydroxybutyronitrile, 2-amino-4-methylmercaptobutyronitrile, 2-amino-2-phenylacetonitrile or 2-amino-3-phenylpropyonitrile, respectively.

### EXAMPLE

Hereinafter, the present invention will be described in detail by way of Example and Comparative Example, however, the present invention is not limited to those examples.

### Example 1

### 1) Culture of Rhodococcus opacus, purification of enzyme, and substrate specificity of enzyme

5 mL of a medium containing 1.0% ofpolypepton, 0.5% of yeast extract and 1.0% of NaCl is placed in a test tube, and sterilized, and then Rhodococcus opacus 71D (FERM AP-21233 or International deposit No. FERM BP-10952) was inoculated to conduct pre-culture at 30°C for 24 hours. 500 mL of a medium containing 0.2% of K₂HPO₄, 0.1% of NaCl, 0.02% of MgSO₄·7H₂O, 0.001% of CaCl₂, 0.05% of polypepton, 0.1% of trace mineral mixture solution and 0.3% of butyronitrile was placed in a 2L Sakaguchi flask, and sterilized, and then the pre-culture solution was inoculated, and main culture was conducted at 30°C for 72 hours at 96 rpm. Meanwhile, the composition of components contained in 1L of the trace mineral mixture solution was as follows: 0.01 g of ZnSO₄·7H₂O, 0.001g of CuSO₄·5H₂O, 0.001 g of MnSO₄·5H₂O, 0.01 g of FeSO₄·7H₂O, 0.01 g of Na₂MoO₄·2H₂O and 0.01 g of CoCl₂·6H₂O.

10 L of the culture solution was centrifuged at 15,000 G for 5 minutes at 4°C to obtain wet cells, was washed with a 10 mM phosphate buffered physiological saline solution with pH 7.0 followed by washing with a 10 mM potassium phosphate buffer with pH 7.0 containing 0.001% of CoCl₂, 0.001% of FeSO₄ and 0.05% of n-butyric acid, and then was resuspended in a 230 mL of the buffer having the same composition. The suspension was treated by ultrasonication for 10 min. twice followed by centrifugation at 15,000 G for 10 minutes at 4°C to obtain a supernatant as a cell-free extract.

Ammonium sulfate was added to the cell-free extract to cause 30% saturation followed by stirring, and then was centrifuged at 20,000 G for 20 minutes at 4°C. The resulting precipitate was dissolved in a small amount of 10 mM potassium phosphate buffer with pH 7.0 containing 0.001% of CoCl₂, 0.001% of FeSO₄ and 0.05% of n-butyric acid, and the resultant solution was subjected to dialysis using the buffer having the same composition to obtain an ammonium sulfate fractionated crude enzyme solution.

The ammonium sulfate fractionated crude enzyme solution was placed in a DEAE-Toyopearl 650M column equilibrated with the buffer having the same composition followed by washing with the buffer having the same composition, and then eluted with the buffer having the same composition with 0→500mM NaCl linear concentration gradient to obtain a DEAE-Toyopearl fractionated crude enzyme solution.

Ammonium sulfate was added to the DEAE-Toyopearl fractionated crude enzyme solution to cause 30% saturation, and the resultant solution was centrifuged at 15,000 G for 10 minutes at 4°C. The supernatant was placed in a Butyl-Toyoperal column equilibrated with 10 mM potassium phosphate buffer with pH 7.0 containing 30% saturated ammonium sulfate, 0.001% of CoCl₂, 0.001% of FeSO₄ and 0.05% of n-butyric acid, followed by washing with the buffer having the same composition containing 20% saturated ammonium sulfate, and then eluted with 20→0% linear concentration gradient of saturated ammonium sulfate, and subj ected to dialysis using the buffer having the same composition containing no ammonium sulfate, to obtain a Butyl-Toyoperal fractionated crude enzyme solution.

The Butyl-Toyoperal fractionated crude enzyme solution was placed in a Gigapite column equilibrated with the buffer having the same composition, followed by washing with the buffer having the same composition, and eluted with 0→0.3M linear concentration gradient of the potassium phosphate buffer, and subjected to dialysis using the buffer having the same composition, to obtain a Gigapite fractionated crude enzyme solution.

The Gigapite fractionated crude enzyme solution was placed in a MonoQ 5/5 column equilibrated with the buffer having the same composition, followed by washing with a 200 mM potassium phosphate buffer containing 0.001% of CoCl₂, 0.001% of FeSO₄ and 0.05% of n-butyric acid, and then eluted with 200→400 mM NaCl linear concentration gradient using the buffer having the same composition, to obtain a MonoQ fractionated crude enzyme solution.

The MonoQ fractionated crude enzyme solution was placed in a hydroxyapatite column equilibrated with 10 mM potassium phosphate buffer with pH 7.0 containing 0.001% of CoCl₂, 0.001% of FeSO₄ and 0.05% of n-butyric acid, followed by washing with the buffer having the same composition, and then eluted with 0→0.3M potassium phosphate buffer linear concentration gradient, and subjected to dialysis using the buffer having the same composition, to obtain a hydroxyapatite- purified enzyme solution.

A variety of aminonitriles were used as substrates, and subjected to enzymatic reaction at 30°C in 0.1 M potassium phosphate buffer with pH 7.0, and the produced amino acid amide was quantitatively determined with HPLC to examine an enzymatic activity for the variety of substrates. The relative activity for each substrate supposing that an activity for 2-aminobutyronitirle was 100 was shown in Table 2.

**Table 2**

| Substrate (20 mM) | Relative activity |
|---|---|
| Alaninonitrile | 77.0 |
| 2-aminobutyronitrile | 100 |
| Valinonitrile | 15.1 |
| t-leuicinonitrile | 1.8 |
| Phenylglycinonitirle | 177 |
| Phenylalaninonitrile a) | 42.9 |
| a) substrate concentration | 10 mM |

### 2) Culture of a bacterium producing aminocaprolactam racemase derived from Achromobacter obae and enzyme purification

A recombinant Escherichia coli JM109/pACL60 having a gene which codes aminocaprolactam racemase derived from Achromobacter obae was cultured at 37°C for 12 hours in accordance with the method disclosed in the Non-Patent Document 1, that is, in an LB medium (triptone 1%, yeast extract 0.5%, NaCl 1% and pH 7.2) with a final concentration of 0.5 mM IPTG and a final concentration of 80 µg/mL ampicillin, and a purified enzyme solution was obtained similarly in accordance with the method disclosed in the Non-Patent Document 1.

### 3) Culture of a bacterium producing D-aminopeptidase derived from Ochrobactrum anthropi and enzyme purification

A recombinant Escherichia coli JM109/pC138DP having a gene which codes a D-aminopeptidase derived from Ochrobactrum anthropi was cultured at 37°C for 12 hours in accordance with the method disclosed in the Non-Patent Document 3, that is, in an LB medium with a final concentration of 1.0% glycerin, a final concentration of 2mg/L thiamine hydrochloride and a final concentration of 50 µg/mL ampicillin, and a purified enzyme solution was obtained similarly in accordance with the method disclosed in the Non-Patent Document 3.

### 4) A production of (R)-2-aminobutyric acid (D-2-aminobutyric acid) from (R,S)-2-aminobutyronitrile using a purified enzyme

1 mL of 0.1 M potassium phosphate buffer at pH 7.0 containing 20 mM (R,S)-2-aminobutyronitrile·1/2 sulfate, 500 nM pyridoxal phosphate, 3.0 U of the above purified enzyme solution derived from Rhodococcus opacus, 1.6 U of the above purified enzyme solution of aminocaprolactam racemase derived from Achromobacter obae, and 0.52 U of the above purified enzyme solution of D-aminopeptitase derived from Ochrobactrum anthropi was allowed to react at 30°C. Analysis of the reaction solution by HPLC revealed that (R)-2-aminobutyric acid (D-2-aminobutyric acid) was produced almost quantitatively in 6 hours. In this instance, no production of (S)-2-aminobutyric acid (L-2-aminobutyric acid) was observed.

### Example 2

### A production of (R)-2-aminobutyric acid (D-2-aminobutyric acid) from (R,S)-2-aminobutyronitrile using acetone powder (an example of change in substrate concentration)

The microorganisms were cultured in the same manner as in Example 1, and acetone powder was prepared in accordance with ordinary method, and used hereinafter in Examples 2-4.

25 mg (18.8 mM) or 50 mg (37.6 mM) of (R,S)-2-aminobutyronitrile·1/2 sulfate was allowed to react at 30°C for 12 hours in 10 mL of 0.1 M potassium phosphate buffer at pH 7.0 containing 500 nM pyridoxal phosphate, 10 mg of acetone powder containing 312 U/g nitrile hydratase derived from Rhodococcus opacus 71D, 50 mg of acetone powder containing 135U/g D-aminopeptitase derived from Ochrobactrum anthropi, and 100 mg of acetone powder containing 175 U/g aminocaprolactam racemase derived from Achromobacter obae. Analysis of the reaction solution by HPLC revealed that (R)-2-aminobutyric acid (D-2-aminobutyric acid) was produced in the yield of 85.9% for 25 mg and 69.2% for 50 mg.

### Example 3

### A production of (R)-2-aminobutyric acid (D-2-aminobutyric acid) from (R,S)-2-aminobutyronitrile using acetone powder (an example of change in reaction temperature)

50mg (37.6 mM) of (R,S)-2-aminobutyronitrile-1/2 sulfate was allowed to react in 10 mL of 0.1 M potassium phosphate buffer at pH 7.0 containing 500 nM pyridoxal phosphate, 0.1 g of acetone powder of containing 312 U/g nitrile hydratase derived from Rhodococcus opacus 71D, 0.2 g of acetone powder containing 135U/g D-aminopeptitase derived from Ochrobactrum anthropi, and 0.3 g of acetone powder containing 175 U/g aminocaprolactam racemase derived from Achromobacter obae at a reaction temperature of 20°C or 30°C for 12 hours. Analysis of the reaction solution by HPLC revealed that (R)-2-aminobutyric acid (D-2-aminobutyric acid) was produced in the yield of 95.7% at a reaction temperature of 20°C and 79.0% at 30°C.

### Example 4

### A production of (R)-2-aminobutyric acid (D-2-aminobutyric acid) from (R,S)-2-aminobutyronitrile using acetone powder (an example of change in reaction pH)

50mg (37.6 mM) of (R,S)-2-aminobutyronitrile·1/2 sulfate was allowed to react in 10 mL of 0.1 M potassium phosphate buffer at pH 6.0 or pH 7.0 containing 500 nM pyridoxal phosphate, 0.05 g of acetone powder containing 312 U/g nitrile hydratase derived from Rhodococcus opacus 71D, 0.1 g of acetone powder containing 135U/g D-aminopeptitase derived from Ochrobactrum anthropi, and 0.1 g of acetone powder containing 175 U/g aminocaprolactam racemase derived from Achromobacter obae at 30°C for 12 hours. Analysis of the reaction solution by HPLC revealed that (R)-2-aminobutyric acid (D-2-aminobutyric acid) was produced in the yield of 62.0% at pH 6.0 and 88.9% at pH 7.0.

### Example 5

### 1) Culture an L-amino acid amidase producing bacterium derived from Brevundimonas diminuta and enzyme purification

A recombinant Escherichia coli JM109 having a gene which codes an L-amino acid amidase derived from Brevundimonas diminuta was cultured at 37°C for 12 hours in accordance with the method disclosed in the Non-Patent Document 4, that is, in an LB medium with a final concentration of 80 µg/mL ampicillin and a final concentration of 0.5 mM isopropyl-β-D-thiogalactopyranoside, and a purified enzyme solution was obtained similarly in accordance with the method disclosed in the Non-Patent Document 4.

### 2) Production of (S)-2-aminobutyric acid (L-2-aminobutyric acid) from (R,S)-2-aminobutyronitrile using a purified enzyme

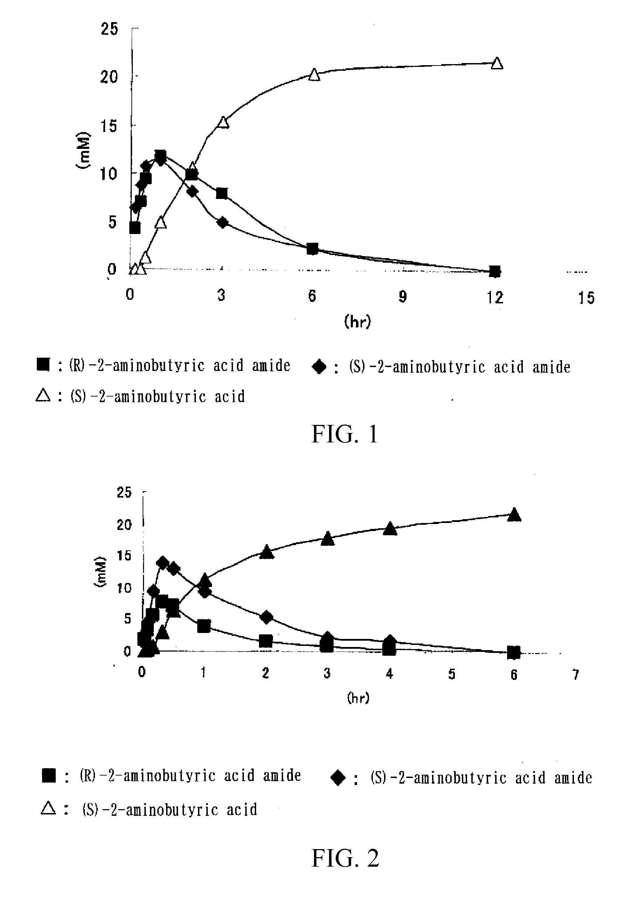

1 mL of 0.1 M potassium phosphate buffer at pH 7.0 containing 20 mM (R,S)-2-aminobutyronitrile·1/2 sulfate, 500 nM pyridoxal phosphate, 0.5 mM CoCl₂, 0.59 U of the above purified enzyme solution derived from Rhodococcus opacus, 1.6 U of the above enzyme solution of aminocaprolactam racemase derived from Achromobacter obae, and 0.39 U of the above purified enzyme solution of L-amino acid amidase derived from Brevundimonas diminuta were allowed to react at 30°C. Analysis of the reaction solution by HPLC revealed that, as shown in Fig. 2, (S)-2-aminobutyric acid (L-2-aminobutyric acid) was produced almost quantitatively in 12 hours. In this instance, no production of (R)-2-aminobutyric acid (D-2-aminobutyric acid) was observed.

### Example 6

### A production of (S)-2-aminobutyric acid (L-2-aminobutyric acid) from (R,S)-2-aminobutyronitrile using acetone powder

The microorganisms were cultured in the same manner as in Example 5, and acetone powder was prepared in accordance with ordinary method, and used in Example 6.

30mg (22.5 mM) of (R,S)-2-aminobutyronitrile·1/2 sulfate was allowed to react at 30°C for 6 hours in 10 mL of 0.1 M potassium phosphate buffer at pH 7.0 containing 500 nM pyridoxal phosphate, 0.5 mM of CoCl₂, 10 mg of acetone powder containing 312 U/g nitrile hydratase derived from Rhodococcus opacus 71D, 50 mg of acetone powder containing 143U/g L-amino acid amidase derived from Brevundimonas diminuta, and 100 mg of acetone powder containing 175 U/g aminocaprolactam racemase derived from Achromobacter obae. Analysis of the reaction solution by HPLC revealed that (S)-2-aminobutyric acid (L-2-aminobutyric acid) was produced in the yield of 77.8%.

### Example 7

### A production of (S)-2-aminobutyric acid (L-2-aminobutyric acid) from (R,S)-2-aminobutyronitrile (Influence of reaction pH)

1 mL of the following buffer at a pH of 5.5 to 9.0 containing 20 mM (R,S)-2-aminobutyronitrile·1/2 sulfate, 500 nM pyridoxal phosphate, 0.5 mM CoCl₂, 8 U of the above purified enzyme solution derived from Rhodococcus opacus, 0.1 U of the above purified enzyme solution of aminocaprolactam racemase derived from Achromobacter obae, and 17 U of the above purified enzyme solution of L-amino acid amidase derived from Brevundimonas diminuta was allowed to react at 30°C for 12 hours. The results of analysis of the reaction solutions by HPLC are shown in the following.

**Table 3**

| pH | Yield (%) of (S)-2-aminobutyric acid |
|---|---|
| 5.5 | 28.9 |
| 6.0 | 42.1 |
| 6.5 | 67.2 |
| 7.0 | 65.2 |
| 7.5 | 76.9 |
| 8.0 | 88.9 |
| 9.0 | 16.8 |

### BRIEF DESCRIPTION FOR THE DRAWINGS

Fig. 1 is a production of (R)-2-aminobutyric acid (D-2-aminobutyric acid) from (R,S)-2-aminobutyronitrile using purified enzymes.
Fig. 2 is a production of (S)-2-aminobutyric acid (L-2-aminobutyric acid) from (R,S)-2-aminobutyronitrile using purified enzymes.

## Claims

1. A method for producing an optically active amino acid composed of a D- or an L-amino acid, which comprises reacting an aminonitrile composed of a mixture of a D-aminonitrile and an L-aminonitrile represented by formula (1) with a biocatalyst which has an activity of converting the two aminonitriles into a D-amino acid amide and an L-amino acid amide respectively, a biocatalyst which has an activity of racemizing the D-amino acid amide and the L-amino acid amide to each other, and a biocatalyst which has an activity of converting one of the D-amino acid amide and the L-amino acid amide into the corresponding D- or L-amino acid. 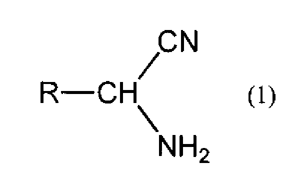 wherein R in the formula (1) is a straight or branched lower alkyl group with 1-4 carbon atoms, a phenyl group or a phenylmethyl group, and may have a hydroxyl group or methylmercapto group as a substituent.

2. The method for producing an optically active amino acid according to claim 1, wherein said biocatalyst having an activity of converting the D-aminonitrile and the L-aminonitrile into a D-amino acid amide and an L-amino acid amide respectively is one derived from a microorganism belonging to the genus Rhodococcus.

3. The method for producing an optically active amino acid according to claim 1, wherein said biocatalyst having an activity of converting the D-aminonitrile and the L-aminonitrile into a D-amino acid amide and an L-amino acid amide respectively is one derived from Rhodococcus opacus.

4. The method for producing an optically active amino acid according to claim 1, wherein said biocatalyst having an activity of racemizing the D-amino acid amide and the L-amino acid amide to each other is one derived from a microorganism belonging to the genus Achromobacter.

5. The method for producing an optically active amino acid according to claim 1, wherein said biocatalyst having an activity of racemizing the D-amino acid amide and the L-amino acid amide to each other is one derived from Achromobacter obae.

6. The method for producing an optically active amino acid according to claim 1, wherein said biocatalyst having an activity of converting the D-amino acid amide selected from the D-amino acid amide and the L-amino acid amide into the corresponding D-amino acid is one derived from a microorganism belonging to the genus Ochrobactrum.

7. The method for producing an optically active amino acid according to claim 1, wherein said biocatalyst having an activity of converting the D-amino acid amide selected from the D-amino acid amide and the L-amino acid amide into the corresponding D-amino acid is one derived from Ochrobactrum anthropi.

8. The method for producing an optically active amino acid according to claim 1, wherein said biocatalyst having an activity of converting the L-amino acid amide selected from the D-amino acid amide and the L-amino acid amide into the corresponding L-amino acid is one derived from a microorganism belonging to the genus Brevundimonas or the genus Xanthobacter.

9. The method for producing an optically active amino acid according to claim 1, wherein said biocatalyst having an activity of converting the L-amino acid amide selected from the D-amino acid amide and the L-amino acid amide into the corresponding L-amino acid is one derived from Brevundimonas diminuta or Xanthobacter flavus.

10. The method for producing an optically active amino acid according to claim 1, wherein, as biocatalysts, those derived from Rhodococcus opacus and Achromobacter obae and one derived from Ochrobactrum anthropi or Brevundimonas diminuta are used in combination.
